# EUROPEAN PATENT SPECIFICATION

(11) **EP 0 981 393 B1**
(45) Date of publication and mention of the grant of the patent: **09.07.2008**
(21) Application number: 97948206.4
(22) Date of filing: 07.11.1997
(51) Int. Cl.: A61N 5/06

(54) **DEVICE FOR MUCOSAL ABLATION USING LIGHT**
GERÄT ZUR ABLATION DER MUKOSA MITTELS LICHT
DISPOSITIF D'ABLATION DES MUQUEUSES AU MOYEN DE LUMIERE

(30) Priority: 21.11.1996 US 33333 P; 22.07.1997 US 903218
(43) Date of publication of application: 01.03.2000
(73) Proprietor: Boston Scientific Limited, St. Michael, Barbados, West Indies (BB)
(72) Inventor: CROWLEY, Robert, J., Sudbury, MA 01776 (US)
(74) Representative: Greenwood, John David
(86) International application number: PCT/US1997/020367
(87) International publication number: WO 1998/022184

(56) References cited:
- EP-A- 0 629 380
- WO-A-96/07451
- DE-A- 3 023 130
- DE-A- 19 512 518
- DE-C- 888 727

## Description

### Technical Field

This invention relates to a medical device that generates ultraviolet light.

### Background Information

Several cancers start in the mucosal linings of the esophagus, throat, intestine and colon and in the endothelial linings of the uterus, urethra, bladder and other organs, ducts and vessels. Barrett's Esophagus is considered to be a pre-malignant condition (e.g.,displasia or metaplasia) observable as the change in cell structure of the esophagus from normal cells to stomach cells. Progressive columnar cell metaplasia, as shown in Barrett's Esophagus cases, is considered to be a pre-malignant condition that may result in adenocarcinoma. It is currently the opinion of leading practitioners of the endoscopic techniques who diagnose Barrett's esophagus that the etiology of the disease involves the repeated exposure of the esophageal tissue to gastric acids (reflux) caused by splashing of the gastric fluids up into the esophagus. Gastro Esophageal Reflux Disease (GERD) is a separate but related condition that allows excessive exposure of the esophagus to stomach acids. Also observable is the effect of ablation or removal of the Barrett's affected portion of the mucosal lining of the esophagus, which generally results in the formation or regrowth of a neo-mucosa, which comprises mainly normal esophageal cells.

Various techniques to destroy only the mucosal linings without excessive damage to the underlying muscularis (i.e., muscle surface) have been attempted. One attempt involves using a high energy ultrasound field to ablate the mucosal linings. There have been claims that cavitational ablation occurs only at the mucosal layer, however these claims have not been verified. There are also mechanical interventions such as excision and chemical measures such as light enhanced photodynamic therapy (PDT). Excision is slow and may result in bleeding or perforation, and is therefore costly. PDT may be effective, but clinical trials have not yet proven its effectiveness. Also, a drug or an agent must be used in conjunction with the light enhanced therapy, which is a disadvantage of PDT.

It would be desirable to be able to ablate or remove the mucosal linings without having to rely on the application of drugs or to resort to a surgical excision. It would be particularly desirable to provide an ablative energy source that could selectively treat only the mucosal linings and not deeper tissue. It would be even more desirable if the energy from the source could also be selectively applied to the areas where the Barrett's cells are most concentrated for reducing the application of ablative energy to normal cells.

EP-A-0 629 380 discloses a combined balloon and laser therapy catheter for performing angioplasty and inhibiting restenosis. An UV flash lamp is mentioned as an alternative to the laser system.

### Summary of the Invention

The present invention is as claimed in the claims.

The invention features an interventional light device for ablating mucosal linings and endothelial linings using high intensity ultraviolet light. The wavelength range of the ultraviolet light permits ablation of tissue near the tissue surface without destroying tissue underneath the surface layer. In addition, the light device may be prepared at low cost, since the device includes an inexpensive flash lamp as the light source.

The claimed device comprises a balloon catheter having a balloon portion, a slidable interventional device within the balloon catheter, and a flash lamp for generating high intensity ultraviolet light disposed near a distal end of the slidable interventional device and inside the balloon portion.

In one embodiment, the flash lamp is a xenon flash lamp and the housing is substantially transparent. In another embodiment, the light device further includes a housing fo protecting the flash lamp. The housing includes a lenticular pattern on a surface of the housing to focus or diffuse light generated by the flash lamp. An example of a lenticular pattern, which may be formed on a surface of the housing is a fresnel pattern. In yet another embodiment, a fluid is transported to the light device though a lumen of the balloon catheter. The fluid flowing adjacent the flash lamp dissipates heat created by the light device. The distal end of the catheter further includes an aperture to remove some of the fluid from the light device.

The foregoing and other objects, aspects, features, and advantages of the invention will become more apparent from the following description and from the claims.

### Brief Description of the Drawings

In the drawings, like reference characters generally refer to the same parts throughout the different views. Also, the drawings are not necessarily to scale, emphasis instead generally being placed upon illustrating the principles of the invention.
FIG. 1 is a view in partial cross-section of a light device disposed near a distal end of a catheter, parallel to a longitudinal axis of the catheter, useful for understanding the present invention;
FIG. 2 is a perspective view of a light device attached to a slidable interventional device.
FIG. 3 is a plan view of a device according to the present invention and including a light device disposed inside a balloon portion of a balloon catheter, parallel to a longitudinal axis of the catheter.
FIG. 4 is a schematic diagram of a catheter probe with a light device inserted into a patient to the esophagus through an endoscope.

### Description

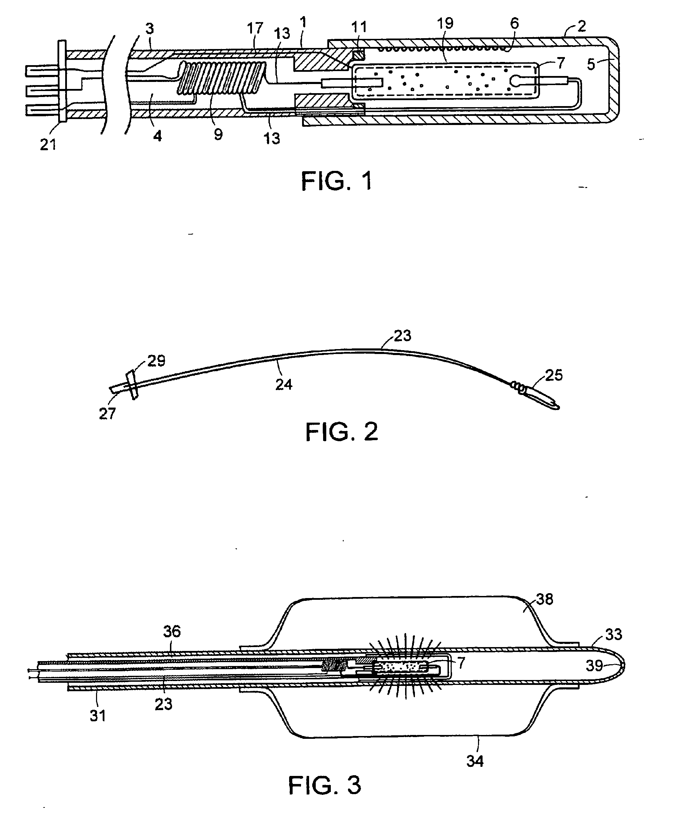

Referring to FIG. 1, a light device 2 includes a housing 5 and a flash lamp 7 placed inside the housing 5. The housing protects the flash lamp 7 and the surrounding anatomy in case of breakage of the flash lamp 7. The housing 5 is adapted for placement inside a body. A flash lamp is a gaseous discharge lamp that produces an output of light of short duration and high intensity. The flash lamp 7 in the light device 2 is capable of generating high intensity ultraviolet light. Various flash lamps can be used successfully in accordance with the invention. In a preferred embodiment, the flash lamp is a xenon flash lamp.

The light device 2 is disposed near a distal end of an interventional device 1. As shown in FIG. 1, the light device 2 is disposed near a distal end of a catheter probe 1. The catheter probe 1 includes a catheter body 3, and the body 3 includes one or more lumens 4. The catheter body 3 is typically made of a single or multi-lumen plastic extrusion of a flexible, resinous and biocompatible material such as nylon, polyethylene or PET. The housing 5 of the light device 2 is at least partially transparent to light. Examples of materials suitable to form the transparent housing 5 include, but are not limited to, polystyrene, polyethylene, and quartz glass. In one embodiment, the housing is attached to the catheter body 3 with an adhesive. In another embodiment, the housing 5 is an extension of the catheter body 3, where the material for the catheter body 3 is optically transparent to light waves. Lenticular or fresnel patterns 6 may be embossed or molded on either surface of the housing 5 to focus or diffuse the light energy generated by the flash lamp 7. In the arrangement of FIG. 1, the flash lamp 7 is secured and centered inside the housing 5 by a friction ring 11.

The light device 2 also includes a pair of leads 13 extending from a voltage source (not shown) to the flash lamp 7. The leads deliver voltage to opposite ends of the flash lamp 7 to cause the flash lamp 7 to generate light. The leads 13 may be connected to a transformer 9, which serves as a voltage step up system for power supplied. Construction of a transformer 9 is well known in the art. The transformer 9, for example, may be constructed by winding a copper wire around a form and tapping the coil at various points to obtain a step up or step down transformer function. In a preferred embodiment, the transformer 9 has a diameter of less than about 0.318 cm (0.125 inches). A small transformer 9 may be used with the light device 2, because the flash light 5 generates light waves with only short duration. When the transformer 9 is placed inside the lumen 4 of the catheter body 3, the transformer 9 may be cooled by surrounding the transformer 9 with a fluid flowing inside the lumen 4. Leads 13 may deliver voltage to the flash light 7 without the transformer 9. For example, small copper wires may be used as the leads 13 so long as the copper wires are insulated sufficiently to prevent arcing. For a 30 gage copper wire, a polytetrafluoroethylene (PTFE) extruded insulation with about 0.001 inch thickness may be suitable.

Still referring to FIG. 1, a third lead 17 is in communication with the flash lamp 7. The third lead 17 is carried through the catheter body 3 and terminates where the lead 17 contacts a small piece of copper foil 19 disposed adjacent a surface of the flash lamp 7. The copper foil 19 aids in the firing of the flash light 7 by providing a higher trigger voltage. Alternatively, in place of a separate lead 17, a thin layer or strip of metalization is deposited on the catheter body 3 and, in place of the copper foil 19, a portion of a surface of the flash lamp 7 is metalized. The metalization on the flash lamp 7 may act as an efficient reflector, redirecting some of the light energy as the operator may desire. A proximal connector 21 located at a proximal end of the catheter 1 provides terminals for leads 13, 17. The proximal connector 21 is in communication with a mating connector for empowering the light device 2. In a preferred embodiment, the connector 21 is a swivel connector that allows the operator to torque the catheter 1 without hindrance.

Referring to FIG. 2, a light device 25 including a transformer is disposed at a distal end of a slidable interventional device 23. The slidable interventional device 23 has an elastic tubing 24 with a length of about 200 cm. In the illustrated arrangement the outside diameter of the light device 25 is about 0.226 cm (0.089 inches), the outside diameter of the elastic tubing 24 is about 0.102 cm (0.040 inches), and the inside diameter is about 0.064 cm (0.025 inches). The elastic tubing may be a super-elastic metal such as nitinol. The proximal connector 27 disposed at a proximal end of the interventional device 23 employs a sliding stop 29. The slidable stop 29 is capable of sliding along the length of the interventional device 23 to control the depth of insertion of the interventional device 23 inside a body. Examples of other materials suitable to form the elastic tubing 24 include, but are not limited to plastic, stainless steel, and composite fiber.

Referring to FIG. 3, the slidable interventional device 23 and the light device 25 of FIG. 2 are inserted inside a catheter 31. The body of the catheter 33 may be made of, for example, a flexible plastic material that is transparent to light. In the embodiment of the present invention disclosed in FIG. 3, a balloon 34 is disposed over the catheter body 33 to provide a space between the flash lamp 7 and tissue to be illuminated. The space provided by the balloon 34 may prevent the tissue from burning. In one embodiment, a fluid is transported through a lumen 36 to the balloon 34 to inflate the balloon 34. Examples of fluids sufficient to inflate the balloon 34 include, but are not limited to, air, water, saline, and radiographic contrast fluid.

The fluid performs an additional function of dissipating heat generated by the flash lamp 7. Higher outputs from the flash lamp 7 are obtainable if waste heat is efficiently removed from the flash lamp 7. Passage of the fluid across the surface of the flash lamp 7 may conveniently remove the waste heat. The catheter 31 further includes an aperture 39 located at the distal end of the catheter 31. In one embodiment, the aperture 39 allows a small amount of the heat dissipating fluid to flow through the catheter 31 past the flash light 7, and to leave the catheter 31 as a way of dissipating heat. In an alternative embodiment, the fluid is re-circulated to and away from the flash light 7 to dissipate heat. However, it has been found that simply surrounding the flash lamp 7 with a cooling fluid is sufficient to reduce heat buildup, thereby allowing higher flash power, flash duration and repetition rate. Repetition rates exceeding about 10 Hz are possible with adequate cooling of the flash lamp 7. The pressure of the cooling fluid may be regulated by a syringe external to a patient. Temperature measurements of the cooling fluid may be taken.

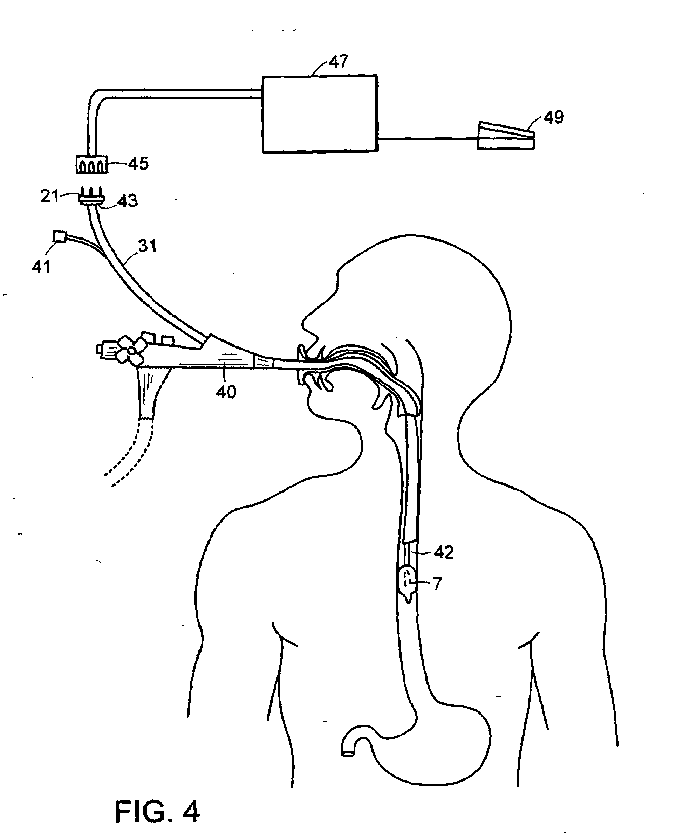

Referring to FIG. 4, the light device 25 and the balloon catheter 31 of FIG. 3 are shown introduced inside an esophagus 42 of a patient through an endoscope 40. The catheter 31 is first prepared by placing the slidable interventional device 23 and the light assembly 25 shown in FIG. 2 inside the catheter 31. The catheter 31 is then introduced inside the body and placed near tissue to be illuminated through the endoscope 40. At this time, a small amount of fluid may be introduced through a luer fitting 41 to wet the catheter and to expel air bubbles from the balloon. A proximal seal 43 located at the proximal end of the catheter 31 prevents fluid from exiting the catheter 31 past the proximal connector 21, where it might cause a short circuit. The proximal connector 21 is connected to the system connector 45, which mates with and receives the proximal connector 21. The system connector 45 is in communication with a control unit 47. The control unit supplies power to the flash lamp 7 when actuated by a foot switch 49. In one embodiment, the control unit 47 includes a capacitor charging circuit and discharging circuit as commonly found in photo flash applications and one or more batteries. In an alternative embodiment, the control unit 47 is equipped with a power supply that is connected to a main outlet through an isolation transformer.

The light device of the invention may be used to illuminate tissue inside a body to serve any number of purposes. In operation, the light device 2 is inserted inside a body near tissue to be illuminated. The light device is then energized with an external power source to generate high intensity ultraviolet light. The tissue is illuminated by applying the generated light to the tissue.

In one method, which does not form part of the present invention, the light device performs ablation of mucosal linings. Application of high intensity ultraviolet light ablates mucosal linings without damaging tissue underneath the linings such as the muscularis, because ultraviolet component of the light is greatly attenuated by tissue. Ultraviolet light is absorbed through only a short distance before it is converted to heat. Therefore, ultraviolet light is particularly effective in destroying the top-most layer of cells, which is the target in ablation of mucosal linings.

This ablation procedure may be carried out by selectively applying the ablative energy to diseased tissue regions only. Tissue under suspicion may be characterized visually, electronically, or optically before being ablated. For example, it is well known that Barrett's esophagus appears pinkish in color and normal esophageal tissue appears whitish in color. The ablation procedure may be carried out using this chromatic or spectral change as a guide. For example, an endoscope 40 is first inserted inside a patient to identify, through a color change, the region of the esophagus that is affected with Barrett's disease. Once the diseased tissue region is identified, a catheter 31 having a light device 2 at the distal end is introduced inside the body through the working channel of the endoscope 40, and appropriately positioned under endoscopic guidance to ablate the diseased tissue. The light device 2 is then energized by an external power supply, and the generated light is applied to a selected region of the body to ablate.

Application of a dye may further enhance the color differences between normal and diseased tissue. The dye may be sprayed to suspicious tissue under pressure through an aperture 39 located at the distal end of an interventional device as shown in FIG. 3. The fact that some dyes may be more absorptive in certain regions of the spectrum and also have a greater affinity to the diseased or normal portions of the tissue may be used for selectively ablating only the diseased tissue. For example, an indigo carmine dye is sprayed onto an area having both diseased and normal tissue. The indigo carmine 0.08% dye, which is blue or violet in color, reflects ultraviolet radiation. The indigo carmine dye also has an affinity for metaplastic tissue, and thus stains the metaplastic tissue to a greater degree and stains the normal tissue to a lesser degree. Therefore, when the light wave energy is applied to tissue stained with the indigo carmine dye, the tissue selectively admits the red, infrared or "heat" energy to a greater degree in the diseased tissue, where it may have a therapeutic effect, but tends to leave the normal tissue relatively unchanged. Alternatively if it is preferred that the diseased tissue absorb the ultraviolet portion of the spectrum, then a red-reflecting dye may be used to stain the tissue under investigation. Any color agent which possesses properties described above may be employed in accordance with the invention. As an alternative arrangement the flash lamp 7 may be coated with a color filter to attenuate the non-ultraviolet portion of the light output from the flash lamp 7.

The use of a flash lamp 7 as a source of high intensity ultraviolet light energy placed in close proximity to tissue region of interest eliminates the need for lasers and light guides. Laser systems are not ideal since light guides tend to attenuate the ultraviolet region of the spectrum and laser systems require very expensive support electronics.

The invention can use ordinary flash electronics such as those found in disposable flash-equipped film cameras, and thus the entire power unit may be discarded after use in an economical manner. The present light device is capable of generating high intensity light in the ultraviolet region, in addition to the visible and infrared regions of the light spectrum. The generated light is applied to various parts of a body for multiple purposes including ablating tissue, heating, crosslinking, activating a drug introduced near the tissue, and/or observing a spectral response of tissue. Some apparatus and methods for observing spectral responses of tissue are disclosed in WO-A-98 22805. Other ultraviolet light sources and methods of using ultraviolet light for diagnostic and therapeutic purposes are described in WO-A-98 22034.

The interventional device of the present invention may be operated by a physician who physically manipulates the device and activates the energy source or remotely controls it under visual guidance and with electronic remote control of the device. The light device may be placed near tissue to be illuminated or actually contact the tissue, such that selected areas of the tissue may burn as a result of the application of the ablation energy. In addition, the light energy may be applied to tissue over a long period of time (for example, longer than a few seconds) such that exposure to radiation is built up over time. The light device may be implanted inside a body for the treatment of tumors that may require prolonged exposure to light.

Variations, modifications, and other implementations of what is described herein will occur to those of ordinary skill in the art without departing from the scope of the invention as claimed. Accordingly, the invention is to be defined not by the preceding illustrative description but instead by the following claims.

## Claims

1. A medical device, comprising:
a balloon catheter (31) having a balloon portion (34), a proximal end, and a distal end; a slidable interventional device (23) within the balloon catheter (31); and
a flash lamp (7) for generating high intensity ultraviolet light disposed near a distal end of the slidable interventional device (23) and inside the balloon portion.

2. The device of claim 1 wherein the flash lamp is a xenon flash lamp.

3. The device of claim 1 further comprising a substantially transparent housing (15).

4. The device of claim 3 wherein the housing includes a lenticular pattern on a surface of the housing to focus or diffuse light generated by the flash lamp.

5. The device of claim 4 wherein the lenticular pattern is a fresnel pattern.

6. The device of claim 1 further comprising a transformer (9) in electrical communication with the flash lamp.

7. The device of claim 1 wherein the balloon catheter has a lumen (36) for transporting a fluid to the balloon portion.

8. The device of claim 7 wherein the balloon catheter has an aperture (39) at the distal end of the catheter for removing the fluid.

9. The device of claim 1 further comprising a sliding stop (29) disposed at a proximal end of the slidable interventional device (23) for controlling depth of insertion of the slidable interventional device.

10. The device of claim 1 further comprising a filter disposed near the distal end of the slidable interventional device (23) for attenuating non-ultraviolet light generated by the flash lamp.

11. The device of claim 1 further comprising a control unit in communication with the flash lamp.

12. The device of claim 1 further comprising a pair of copper wires insulated with polytetrafluoroethlyene as leads (13) in electrical communication with the flash lamp.

13. The device of claim 12, further comprising:
a third lead (17) in contact with a copper foil (19) disposed adjacent a surface of the flash lamp (7).

14. The device of claim 13, further comprising:
a swivel connector (27) disposed at a proximal end of the slidable interventional device (23).

## Patentansprüche

1. Medizinische Vorrichtung, mit:
einem Ballonkatheter (31), der einen Ballonabschnitt (34), ein proximales Ende und ein distales Ende aufweist;
einer verschiebbaren Interventionsvorrichtung (23) innerhalb des Ballonkatheters(31); und
einer Blitzlampe (7) zum Erzeugen von UV-Licht hoher Intensität, die nahe einem distalen Ende der verschiebbaren Interventionsvorrichtung (23) und im Innern des Ballonabschnitts angeordnet ist.

2. Vorrichtung gemäß Anspruch 1, bei der die Blitzlampe eine Xenon-Blitzlampe ist.

3. Vorrichtung gemäß Anspruch 1, ferner mit einem im Wesentlichen transparenten Gehäuse (15).

4. Vorrichtung gemäß Anspruch 3, bei der das Gehäuse ein Linsenmuster auf einer Oberfläche des Gehäuses aufweist, um durch die Blitzlampe erzeugtes Licht zu fokussieren oder zu zerstreuen.

5. Vorrichtung gemäß Anspruch 4, bei der das Linsenmuster ein Fresnelmuster ist.

6. Vorrichtung gemäß Anspruch 1, ferner mit einem Transformator (9) in elektrischer Kommunikation mit der Blitzlampe.

7. Vorrichtung gemäß Anspruch 1, bei der der Ballonkatheter ein Lumen (36) zum Transportieren eines Fluids zu dem Ballonabschnitt aufweist.

8. Vorrichtung gemäß Anspruch 7, bei der der Ballonkatheter eine Apertur (39) an dem distalen Ende des Katheters zum Entfernen des Fluids aufweist.

9. Vorrichtung gemäß Anspruch 1, ferner mit einem Anschlagschieber (29), der an einem proximalen Ende der verschiebbaren Interventionsvorrichtung (23) zum Steuern der Einführungstiefe der verschiebbaren Interventionsvorrichtung angeordnet ist.

10. Vorrichtung gemäß Anspruch 1, ferner mit einem Filter, das nahe dem distalen Ende der verschiebbaren Interventionsvorrichtung (23) zum Abschwächen von durch die Blitzlampe erzeugtem Nicht-UV-Licht angeordnet ist.

11. Vorrichtung gemäß Anspruch 1, ferner mit einer Steuereinheit in Kommunikation mit der Blitzlampe.

12. Vorrichtung gemäß Anspruch 1, ferner mit einem Paar von mit Polytetrafluoroethylen isolieren Kupferdrähten als Leitungen (13) in elektrischer Kommunikation mit der Blitzlampe.

13. Vorrichtung gemäß Anspruch 12, ferner mit:
einer dritten Leitung (17) in Kontakt mit einer Kupferfolie (19), die benachbart zu einer Oberfläche der Blitzlampe (7) angeordnet ist.

14. Vorrichtung gemäß Anspruch 13, ferner mit:
einem Schwenkverbinder (27), der an einem proximalen Ende der verschiebbaren Interventionsvorrichtung (23) angeordnet ist.

## Revendications

1. Dispositif médical, comportant :
un cathéter à ballonnet (31) ayant une partie de ballonnet (34), une extrémité proximale et une extrémité distale, un dispositif d'intervention coulissant (23) dans le cathéter à ballonnet (31), et
une lampe flash (7) pour générer de la lumière ultraviolette haute intensité, disposée à proximité d'une extrémité distale du dispositif d'intervention coulissant (23) et à l'intérieur de la partie de ballonnet.

2. Dispositif selon la revendication 1, dans lequel la lampe flash est une lampe flash au xénon.

3. Dispositif selon la revendication 1, comportant en outre un boîtier sensiblement transparent (15).

4. Dispositif selon la revendication 3, dans lequel le boîtier comprend un motif lenticulaire sur une surface du boîtier pour focaliser ou diffuser une lumière générée par la lampe flash.

5. Dispositif selon la revendication 4, dans lequel le motif lenticulaire est un motif de Fresnel.

6. Dispositif selon la revendication 1, comportant en outre un transformateur (9) en communication électrique avec la lampe flash.

7. Dispositif selon la revendication 1, dans lequel le cathéter à ballonnet a une lumière (36) pour transporter un fluide vers la partie de ballonnet.

8. Dispositif selon la revendication 7, dans lequel le cathéter à ballonnet a une ouverture (39) au niveau dé l'extrémité distale du cathéter pour enlever le fluide.

9. Dispositif selon la revendication 1, comportant en outre une butée coulissante (29) disposée à une extrémité proximale du dispositif d'intervention coulissant (23) pour commander la profondeur d'insertion du dispositif d'intervention coulissant.

10. Dispositif selon la revendication 1, comportant en outre un filtre disposé à proximité de l'extrémité distale du dispositif d'intervention coulissant (23) pour atténuer une lumière non-ultraviolette générée par la lampe flash.

11. Dispositif selon la revendication 1, comportant en outre une unité de commande en communication avec la lampe flash.

12. Dispositif selon la revendication 1, comportant en outre une paire de fils de cuivre isolés par du polytétrafluoroéthylène en tant que fils conducteurs (13) en communication électrique avec la lampe flash.

13. Dispositif selon la revendication 12, comportant en outre :
un troisième fil conducteur (17) en contact avec une feuille de cuivre (19) disposée adjacente à une surface de la lampe flash (7).

14. Dispositif selon la revendication 13, comportant en outre :
un raccord pivotant (27) disposé à une extrémité proximale du dispositif d'intervention coulissant (23).
